(19) Europäisches Patentamt
European Patent Office
Office européen des brevets

(11) Veröffentlichungsnummer : **0 130 224**
**B1**

(12) # EUROPÄISCHE PATENTSCHRIFT

(45) Veröffentlichungstag der Patentschrift : **10.09.86**

(51) Int. Cl.[4] : **C 07 C 87/60**, C 07 C109/10, C 07 C143/825, C 07 C119/10

(21) Anmeldenummer : **83106350.8**

(22) Anmeldetag : **29.06.83**

(54) **Verfahren zur Herstellung von 2-Amino-3,5-dibrombenzylaminen.**

(43) Veröffentlichungstag der Anmeldung : **09.01.85 Patentblatt 85/02**

(45) Bekanntmachung des Hinweises auf die Patenterteilung : **10.09.86 Patentblatt 86/37**

(84) Benannte Vertragsstaaten : **AT BE DE FR GB IT NL**

(56) Entgegenhaltungen : **DE-A- 2 218 647**

(73) Patentinhaber : **LUDWIG HEUMANN & CO GMBH**
**Heideloffstrasse 18-28 Postfach 2260**
**D-8500 Nürnberg (DE)**

(72) Erfinder : **Liebenow, Walter, Dr.**
**Ganghoferstrasse 23**
**D-8500 Nürnberg 21 (DE)**
Erfinder : **Grafe, Ingomar, Dr.**
**Auerbacher Strasse 18**
**D-8500 Nürnberg 30 (DE)**

(74) Vertreter : **Kraus, Walter, Dr. et al**
**Patentanwälte Kraus, Weisert & Partner Thomas-Wimmer-Ring 15**
**D-8000 München 22 (DE)**

**EP 0 130 224 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 2-Amino-3,5-dibrombenzylaminen der allgemeinen Formel

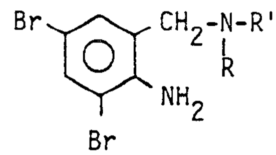

(I)

in der R für Wasserstoff oder $C_{1-3}$-Alkyl steht und R' einen gegebenenfalls substituierten aliphatischen Rest mit 1 bis 5 Kohlenstoffatomen, einen gegebenenfalls substituierten cycloaliphatischen Rest mit 5 oder 6 Kohlenstoffatomen oder eine Phenylgruppe, die gegebenenfalls substituiert ist, bedeutet, sowie deren physiologisch verträgliche Salze mit anorganischen oder organischen Säuren.

In der DE-PS 1 593 579 werden Hydroxycyclohexylamine der allgemeinen Formel

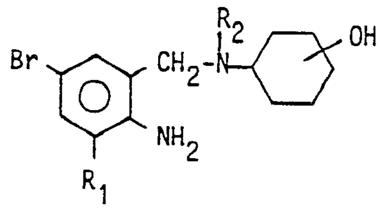

mit wertvollen pharmakologischen Eigenschaften beschrieben, die insbesondere die periphere Atmungsfunktion beeinflussen und ausgezeichnete sekretolytische und hustenstillende Wirksamkeit bei geringer Toxizität aufweisen.

Die wichtigste Verbindung dieser Gruppe ist die Verbindung N-(trans-4-Hydroxy-cyclohexyl)-(2-amino-3,5-dibrom-benzyl)-amin-hydrochlorid (Ambroxol). Auch die am Stickstoff methylierte Verbindung (Cyclohexylaminverbindung) hat als Bromhexin Bedeutung erlangt.

Substanzen dieser Stoffgruppe werden nach der DE-PS 1 593 579 dadurch hergestellt, daß man ein Hydroxycyclohexylamin der allgemeinen Formel

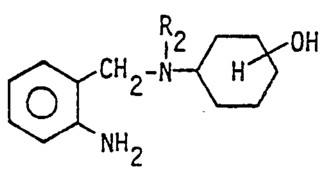

mit Brom in Eisessig oder das Mono- bzw. Diacylaminobenzylhalogenid der allgemeinen Formel

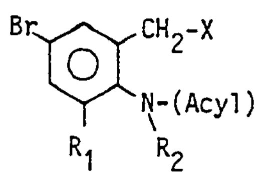

$R_2$ = H oder Acyl

mit einem Aminocyclohexanol umsetzt oder die Verbindung der allgemeinen Formel

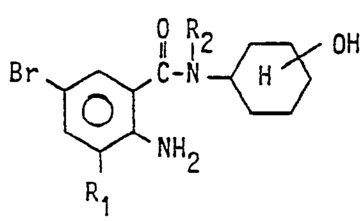

mit Lithiumaluminiumhydrid in Tetrahydrofuran reduziert.

Gemäß der DE-OS 2 345 443 wird eine Verbindung der allgemeinen Formel

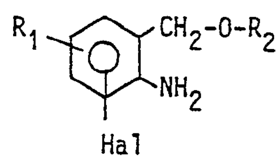

$R_1$ = Wasserstoff- oder Bromatom
$R_2$ = Methyl oder Phenyl
mit einem Amin umgesetzt. Bei dem Verfahren der DE-OS 2 402 577 wird eine Verbindung der allgemeinen Formel

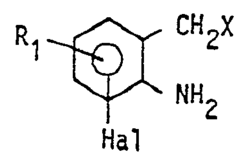

in der X einen Trialkylammonium- oder Pyridiniumrest darstellt, mit einem Amin zu der angestrebten Verbindung umsetzt.

Bei diesen Verfahren muß zur Herstellung der angestrebten Verbindungen meistens vom o-Aminobenzaldehyd bzw. o-Nitrobenzaldehyd ausgegangen werden. Wegen der Neigung zur Selbstkondensation und der nicht guten Handhabung der o-Nitrobenzaldehyde (exotherme Zersetzung) als Ausgangsmaterial für die entsprechenden Aminobenzaldehyde sind diese Verfahren daher nicht befriedigend. Es sind daher weitere Verfahren vorgeschlagen und zum Patent angemeldet worden. Aber auch diese Verfahren sind hinsichtlich der Einfachheit der Durchführung und der erhältlichen Ausbeute nicht in jeder Hinsicht zufriedenstellend.

Aufgabe der Erfindung ist es daher, ein vereinfachtes und wirtschaftlich günstiges Verfahren zur Herstellung der oben erwähnten Verbindungen zur Verfügung zu stellen, das von den oben erwähnten Mängeln frei ist und durch das die angestrebten Verbindungen in einfacher Weise und mit guter Ausbeute erhalten werden können.

Diese Aufgabe wird erfindungsgemäß bei einem Verfahren der eingangs erwähnten Art dadurch gelöst, daß man

a) einen Anthranilsäurealkylester der allgemeinen Formel

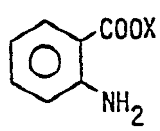

(II)

worin X für eine Niedrigalkylgruppe steht, in Gegenwart eines Oxidationsmittels zu dem 3,5-Dibromanthranilsäureester III

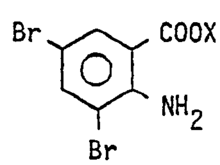

(III)

bromiert,

b) den entstandenen 3,5-Dibromanthranilsäureester III mit Hydrazin zum Hydrazid der allgemeinen Formel

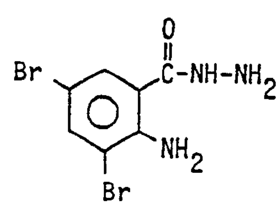

(IV)

umsetzt,

c) das erhaltene Hydrazid IV mit einem Sulfohalogenid zum N-(2-Amino-3,5-dibrombenzyl)-N'-sulfonylhydrazid der Formel

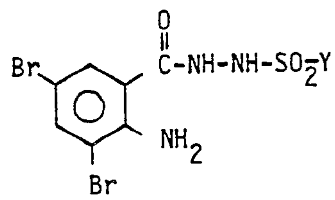

(V)

worin Y ein aliphatischer oder aromatischer Rest ist, umsetzt,

d) das erhaltene Sulfonylhydrazid V im alkalischen Medium mit einem primären Amin der allgemeinen Formel

$$H_2N—R' \quad (VI)$$

worin R' die oben angegebene Bedeutung hat, zu der Schiff'schen Base VII

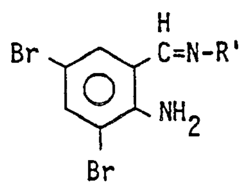

(VII)

umsetzt,

e) die erhaltene Schiff'sche Base VII zu der Verbindung der allgemeinen Formel I hydriert und gegebenenfalls

f) zur Herstellung einer Verbindung, bei der R für $C_{1-3}$-Alkyl steht, mit einem Alkylierungsmittel alkyliert und gegebenenfalls

g) die in Stufe e) oder f) erhaltene Verbindung in an sich bekannter Weise in ihr Salz überführt.

In der allgemeinen Formel I bedeutet R ein Wasserstoffatom oder eine $C_{1-3}$-Alkylgruppe, beispielsweise eine Methyl-, Ethyl-, n- oder Isopropyl- und insbesondere eine Methylgruppe. R' ist ein gegebenenfalls substituierter aliphatischer Rest mit 1 bis 5 Kohlenstoffatomen, beispielsweise eine Methylgruppe, Ethylgruppe, Propylgruppe, Butylgruppe oder Pentylgruppe. Die letztgenannten Gruppen können geradkettig oder verzweigt sein. Diese Alkylgruppe kann durch Halogen-, Amino-, Hydroxy- oder Alkoxygruppen substituiert sein. R' bedeutet weiterhin einen gegebenenfalls substituierten cycloaliphatischen Rest mit 5 oder 6 Kohlenstoffatomen, d. h. einen gegebenenfalls substituierten Cyclopentyl- oder Cyclohexylrest. Der cycloaliphatische Rest kann unsubstituiert sein oder beispielsweise durch eine oder mehrere Hydroxy-, Oxy-, Dioxyalkylen-, Halogen- oder Aminoreste substituiert sein. Vorzugsweise ist der cycloaliphatische Rest unsubstituiert oder mit einer 4-Hydroxygruppe substituiert. Als Dioxyalkylenrest wird der Dioxyethylenrest bevorzugt.

In der ersten Stufe des erfindungsgemäßen Verfahrens wird ein Anthranilsäurealkylester der allgemeinen Formel II

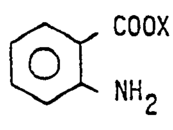

(II)

worin X für eine Niedrigalkylgruppe, vorzugsweise eine Methyl- oder Ethylgruppe, steht, in Gegenwart eines Oxidationsmittels zu dem 3,5-Dibromanthranilsäureester III

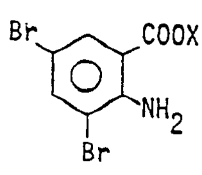

(III)

bromiert. In der Formel III hat X die in der Formel II angegebene Bedeutung. Die Bromierung verläuft in Gegenwart eines Oxidationsmittels, vorzugsweise von Hydroperoxid. Die Umsetzung erfolgt im Temperaturintervall von 20 °C bis zum Siedepunkt des Gemisches aus Wasser und einem mit Wasser nicht mischbaren Lösungsmittel, bevorzugt einem chlorierten Kohlenwasserstoff.

Der erhaltene 3,5-Dibromanthranilsäureester wird in üblicher Weise, beispielsweise durch Kristallisation aus einem niedrigen Alkohol, isoliert und für die nächste Stufe verwendet.

In dieser erfolgt die Umsetzung mit Hydrazin zum Hydrazid der allgemeinen Formel IV

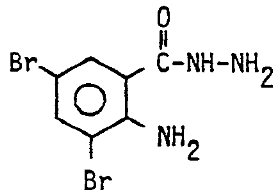

(IV)

Die Umsetzung erfolgt bei folgenden Bedingungen :

Der Anthranilsäureester der Formel III wird in einem mit Wasser mischbaren Alkohol, dessen Siedepunkt über 100 °C liegt, wie z. B. Methylglycol, mit einem Überschuß an Hydrazinhydrat unter Rückfluß gekocht, wobei der entstehende niedrige Alkohol XOH der Formel III abdestilliert werden kann.

Das bei der Reaktion entstandene Hydrazid wird allgemeiner Weise, beispielsweise durch Verdünnen des Reaktionsgemisches mit Wasser, isoliert.

In der nächsten Stufe wird das erhaltene Hydrazid IV mit einem Sulfohalogenid zum N-(2-Amino-3,5-dibrombenzyl)-N'-sulfonylhydrazid der Formel V

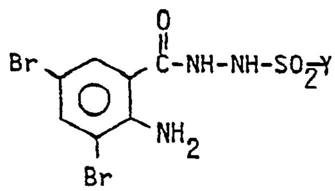

(V)

umgesetzt. In der Formel V ist Y ein aliphatischer oder aromatischer Rest, beispielsweise ein Niedrigalkyl- oder substituierter Phenyl- oder Naphthylrest. In dieser Stufe können die üblicherweise in der synthetischen Chemie verwendeten Sulfohalogenide eingesetzt werden, wobei Methansulfochlorid und Toluolsulfochlorid bevorzugt werden, aber jedoch die erhaltenen Mesylate in der folgenden Stufe bessere Ausbeuten liefern.

Die Umsetzung erfolgt bei 0 bis 100 °C in Gegenwart einer Hilfsbase, wie z. B. Pyridin oder Triäthylamin in einem organischen Lösungsmittel, das die Stoffe genügend löst, wie z. B. Essigester, THF, Dioxan oder 1,2-Dimethoxyethan, und das erhaltene Produkt wird in üblicher Weise aufgearbeitet und isoliert.

Sodann wird das in dieser Stufe erhaltene Sulfonylhydrazid V im alkalischen Medium mit einem primären Amin der allgemeinen Formel VI

$$H_2N—R' \qquad (VI)$$

zu der Schiff'schen Base VII

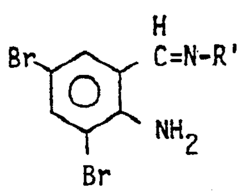

(VII)

umgesetzt. In der Formel VI hat R' die oben angegebene Bedeutung. Nach einer Ausführungsform des erfindungsgemäßen Verfahrens wird ein Amin VI eingesetzt, bei dem R' eine durch eine Dioxyethylengruppe substituierte Cyclohexylgruppe ist. Dieses Aminocyclohexanolketal der Formel VIII

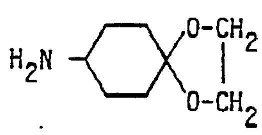

(VIII)

liefert bei der Umsetzung eine Ketalverbindung der Formel IX

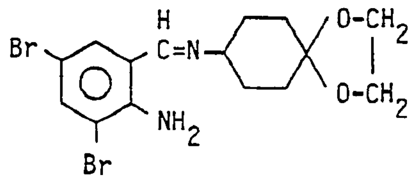

(IX)

die in der darauffolgenden Stufe mit einem Reduktionsmittel hydriert wird.

Die Umsetzung des Sulfonylhydrazids mit dem primären Amin verläuft in einem speziellen Lösungsmittel, nämlich einem Alkohol, vorzugsweise in Methylglykol oder Butanol oder einem Gemisch dieser Alkohole. Es wird im alkalischen Medium, vorzugsweise in Anwesenheit von Kaliumcarbonat, Soda oder einem Alkalihydroxid gearbeitet. Ansonsten verläuft die Umsetzung bei 60 bis 140 °C.

Wird, wie oben erwähnt, als primäres Amin das Aminocyclohexanonketal verwendet, dann wird nach der Umsetzung und Reduktion, die die Ketalverbindung liefern, Säure, beispielsweise Salzsäure oder Schwefelsäure, zugesetzt, damit das Ketal zum Keton gespalten wird.

In der darauffolgenden Stufe wird das erhaltene Keton zu der Verbindung der allgemeinen Formel I hydriert. Als Hydrierungsmittel kommen beispielsweise katalytisch aktivierter Wasserstoff oder Komplexe Hydride in Betracht, wobei Alkaliborhydrid, wie Natriumborhydrid, bevorzugt werden.

Die oben genannten Reduktionsmittel dienen auch zur Reduktion der Schiff'schen Base der Formel VII, wobei entweder in einem niedrigen Alkohol, wie z. B. Methanol oder Äthanol, oder in einem höheren, wie z. B. n-Butanol in Gegenwart von Wasser, wobei zur Aufrechterhaltung eines konstanten pH-Wertes ein Puffer, wie z. B. Natriumbicarbonat oder Trinatriumphosphat, zugesetzt werden kann, gearbeitet werden kann.

Wird die Herstellung einer Verbindung in Betracht gezogen, bei der R eine Alkylgruppe mit 1 bis 3 Kohlenstoffatomen, beispielsweise eine Methylgruppe, ist, dann wird in einer weiteren Stufe die erhaltene Verbindung mit einem Alkylierungsmittel, beispielsweise Dimethylsulfat, in üblicher Weise alkyliert. Die Alkylierung erfolgt beispielsweise in einem Lösungsmittel, wie Wasser oder einem Alkohol, und bei Temperaturen von 10 bis 80 °C. Die Aufarbeitung des erhaltenen Produktes erfolgt in üblicher Weise, beispielsweise durch Destillation der Base oder durch Fällung als Salz, bevorzugt als Hydrohalogenid.

Die bei der Umsetzung erhaltene Verbindung der allgemeinen Formel I kann in an sich bekannter Weise mit einer organischen oder anorganischen Säure in ihr Salz umgewandelt werden. Beispiele für geeignete Salze sind die Hydrochloride, Hydrobromide, Sulfate, Acetate, Maleinate, Maleate, Fumarate, Oxalate, Methansulfonate, Tartrate, Zitrate, Succinate und Embonate etc.

Die Erfindung wird in den Beispielen erläutert.

## Beispiele

### Beispiel 1

Darstellung von 3,5-Dibromanthranilsäuremethylester

In einem 5 l fassenden Rundkolben werden in 2,1 l Dichloräthan 6 Mol Anthranilsäuremethylester (777 ml), 0,48 l Wasser und 6 ml $H_2SO_4$ vorgelegt.

Unter Rühren tropft man in 1 h 6,42 Mol Brom (1,026 kg) bei maximal 70 °C zu und läßt 10 min nachreagieren. Bromanthranilsäuremethylesterhydrobromid fällt aus.

Bei 65-70 °C tropft man unter Kühlung so schnell wie möglich 6 Mol $H_2O_2$ (30-35 %ig) zu, bis ein geringer Überschuß an Brom bestehen bleibt.

Diesen vernichtet man nach 20 min mit $Na_2S_2O_5$, führt eine Phasentrennung durch und zieht das Lösungsmittel im Vakuum ab.

Das Produkt kristallisiert man aus 3 l Methanol und saugt bei 10 °C ab.

Ausbeute : 1,78 kg = 96 % d. Th. ; Fp. 86,2-87,1 °C.

### Beispiel 2

Darstellung von Dibromanthranilsäurehydrazid

In 1 l Methylglycol, 4 Mol 80 % Hydrazinhydrat (246 ml) werden 2 Mol Dibromanthranilsäureester 4 h unter Rückfluß gekocht.

Man saugt bei 20 °C ab und digeriert mit 0,7 l Methanol.

Ausbeute : 509 g = 82,4 % d. Th. ; Fp. 190,3-191,2 °C.

### Beispiel 3

Darstellung von N(3,5)-Dibromanthraniloyl-N'-methansulfonylhydrazid

In 1 l Dioxan und 2,2 Mol Pyridin (177 ml) werden 400 g von 618 g (2 Mol) Dibromanthranilsäurehydrazid vorgelegt.

Unter Rühren tropft man 90 ml von 157 ml Methansulfonylchlorid in 10 min zu, wobei die Innentemperatur bis zum Rückfluß steigt.

Wenn die exotherme Umsetzung nachgelassen hat, werden die Reste an Hydrazid und Mesylchlorid zugegeben. Nach einer Nachreaktionszeit von 30 min bei 80 °C werden 0,6 l Dioxan im Vakuum abgezogen und der Rückstand bei 80 °C mit 0,4 l Methanol und 0,3 l Wasser versetzt.

Man saugt bei 15 °C ab und digeriert mit 1 l Methanol.

Ausbeute : 766 g = 99 % d. Th. ; Fp. 237-243 °C.

## Beispiel 4 (Zweistufen-Variante)

Darstellung von 2-Amino-3,5-dibrombenzyliden-4-trans-hydroxy-cyclohexylamin

In 1,2 l Methylglycol löst man 1,2 Mol NaOH und gibt dann 1,2 Mol 4-trans-Cyclohexanolaminhydrochlorid und 0,8 Mol Pottasche zu. Man heizt auf 110-120 °C und trägt in Portionen 1,2 Mol 3,5-Dibrom-N'-methansulfonylanthranilsäurehydrazid so schnell ein, wie es die Schaumbildung erlaubt. Nach der Zugabe hält man das Gemisch so lange bei 120 °C, bis keine Gase mehr entweichen.

Im Vakuum engt man auf 0,5-0,7 l ein und füllt mit Wasser bei 30-40 °C auf 2 l auf.

Der Niederschlag wird abgesaugt, mit Wasser gewaschen und aus Perchloräthylen nach Klärfiltration umkristallisiert.

Ausbeute : 390 g (gelbe Prismen) = 70 % d. Th.

Fp. 77 °C (Umwandlung), 122,4-124,8 °C

Die Substanz kristallisiert mit einem halben Mol des Lösungsmittels, das sie beim Trocknen im Vakuum abgibt.

| | | | | | | |
|---|---|---|---|---|---|---|
| Ber. : | $C_{14}H_{16}Br_2Cl_2N_2O$ | C 36,63 | H 3,51 | N 6,10 | Hal (als Br) | 58,83 |
| Gef. : | | C 36,57 | H 3,63 | N 6,11 | Br | 61,68 |

In 93 % d. Th. kann man die obige Schiffbase isolieren, wenn man die Mc-Fadyen-Stevens-Umlagerung in Glycol Butanol 1 : 2 in Gegenwart von NaOH durchführt.

Reduktion der Schiffbase zu Ambroxol

1,2 Mol der obigen Schiffbase werden in 0,9 l Methanol gelöst, wobei das Methanoladdukt ausfallen kann, und bei 30-45 °C durch Zugabe von 0,9 Mol Natriumboranat in kleinen Portionen reduziert.

Nach beendeter Reaktion wird das Lösungsmittel abgezogen und der Rückstand heiß zwischen 1 l Perchloräthylen und 0,3 l Wasser verteilt. Beim Abkühlen der org. Phase auf 5 °C fällt Ambroxolbase aus. Sie wird in 0,9 l Aceton mit konz. Salzsäure als Hydrochlorid gefällt und aus 5 Tl. Wasser umkristallisiert.

Ausbeute : 358 g = 80 % d. Th. ; Fp. 232-235 °C

## Beispiel 5

Eintopfvariante zur Darstellung von Ambroxol

In 0,4 l Glycol werden 3 Mol NaOH gelöst und bei 60-90 °C 1,5 Mol 4-trans-cyclohexanolaminhydrochlorid zugegeben.

Nach Zugabe von 0,8 l n-Butanol gibt man die Hälfte von 1,5 Mol 3,5-Dibrom-N'-methansulfonylanthranilsäurehydrazid zu und rührt 1 h bei 110-115 °C. Dann gibt man den Rest des Hydrazids so zu, daß das Gemisch rührfähig bleibt, rührt 3 h bei 110-115 °C und kocht noch 1 h unter Rückfluß. Man kühlt auf 40 °C und füllt mit Wasser auf 2,2 l auf. Die org. Phase wird mit 0,4 l 30 % NaOH gewaschen.

Zur Reduktion löst man in 0,3 l Wasser 18 mMol Äthylendiamintetraessigsäure, 0,10 Mol NaOH und 15 mMol Vanadin(IV)sulfat oder Ammoniumvanadat, gibt die org. Phase hinzu und stellt mit Natriumbicarbonat pH = 11-11,5 ein.

Durch Zugabe von 1,11 Mol Natriumboranat in Portionen erfolgt die exotherme Reduktion, zu deren Beendigung man die Temperatur von 45 auf 80-90 °C steigert.

Die wäßrige Phase wird abgetrennt, das Lösungsmittel abgezogen und der Rückstand wie in Beispiel 4 gereinigt.

Ausbeute : Ambroxol 70 % d. Th.

## Beispiel 6

$N^8$-(2-Amino-3,5-Dibrombenzyliden)-1,4-dioxaspiro[4,5]decylamin ($C_{15}H_{18}Br_2N_2O_2$)

Unter Einhaltung der molaren Mengen des Beispiels 5 unter Einsatz von 1,4-Dioxaspiro 4,5-8-decylamin erhält man obige Verbindung in 80 % d. Th., wenn man das Butanol/Wassergemisch auf 10 °C kühlt und absaugt. Die Schiffbase ist in Butanol wenig löslich.

Fp. 115,0-115,7 °C.

Beispiel 7

$N^8$-(2-Amino-3,5-dibrombenzyl)-1,4 dioxaspiro [4,5] decylamin-Hydrochlorid ($C_{15}H_{21}Br_2ClN_2O_2$)

Unter Einhaltung der Reduktionsbedingungen von Beispiel 4 erhält man obige Verbindung in 80 % d. Th.

Fp. 236,1-237,5 °C
Ber.: C 39,45 H 4,64 Hal 42,77 N 6,14
Gef.: C 39,10 H 4,66 Hal 42,58 N 6,10

Die freie Base erhält man durch Rühren des schwerlöslichen Hydrochlorids in Dichloräthan und verd. wäßrigem Ammoniak. Nach Abtrennung der org. Phase und Abziehen des Lösungsmittels erhält man die freie Base aus Methanol.

Fp. 55,8-57,7 °C.

Beispiel 8

Darstellung von Ambroxol aus $N^8$-(2-Amino-3,5-dibrombenzyl)-1,4-dioxaspiro [4,5] decylamin aus Beispiel 7

210 g der Verbindung aus Beispiel 7 (0,5 Mol) werden in 0,21 l Dichloräthan, 100 ml $H_2O$, 10 ml i-Propanol und 50 ml konz. Ammoniak bei 50 °C 10 min kräftig gerührt. Nach Klärfiltration erfolgt die Phasentrennung. Die org. Phase rührt man 30 min mit 0,3 l Wasser und 50 ml $H_2SO_4$ bei 50-60 °C durch und trennt die org. Phase ab.

Die wäßrige Phase wird mit 0,3 l Perchloräthylen versetzt und mit NaOH (30 %) pH = 11 bis 12,5 eingestellt. Man gibt 10 g Soda zu und reduziert bei 20-30 °C mit 0,15 Mol Natriumboranat nach Zugabe von 10 g Tetrabutylammoniumbisulfat.

Die org. Phase wird bei 80 °C abgetrennt, mit heißem Wasser gewaschen und abgesaugt.

Die Darstellung von Ambroxol aus der Base erfolgt analog Beispiel 4.

Ausbeute : 70 % = 145 g

Beispiel 9

Darstellung von Bromhexin

1. Stufe : 2-Amino-3,5-dibrombenzylidencyclohexylamin
In 0,25 l Glycol werden
0,75 Mol NaOH = 30 g
20 g Soda gelöst bzw. suspendiert. Dazu gibt man
0,75 Mol Cyclohexylamin und
0,25 l n-Butanol und erwärmt auf 80 °C.

Die Hälfte von 0,75 Mol N'-Methansulfonyl-3,5-dibromanthranilsäurehydrazid = 1/2 · 290 g werden zugegeben, und man hält 1 h bei 110 °C, wobei sich der Niederschlag verringert und 7,5 l $N_2$ entweichen. Nach Zugabe der 2. Portion tritt wieder Verdickung ein, jedoch bleibt die Mischung rührfähig.

Nach insgesamt 5,5 h sind 19,3 l Gas entwichen, und man füllt mit Wasser auf 1,1 l auf.

Das Produkt fällt ab 60 °C (ggf. animpfen), und man saugt das zweiphasige Gemisch (Wasser unten) bei RT ab und wäscht mit Wasser und 80 % Methanol.

Ausbeute : 225 g = 84 % d. Th. ; Fp. 81,6-82,6 °C ; gelbe Prismen.

2. Stufe : Reduktion zu 2-Amino-3,5-dibrombenzylcyclohexylamin
In 0,1 l Wasser legt man vor :
5 g Soda.

Dann gibt man 0,4 l n-Butanol, 270 g (0,75 Mol) Schiffbase und portionsweise so viel $NaBH_4$ in 2 h bei max. 45 °C zu, bis keine Wärmetönung mehr feststellbar ist. Der Verbrauch beträgt 29 g.

Man erwärmt auf 80 °C, um Boranat zu zerstören und trennt nach 1/2 h die wäßrige Phase ab.

Man wäscht mit 200 ml 30 % NaOH und 200 ml 10 % NaCl-Lösung.

Nach Zusatz von 5 g $CaCO_3$ wird destilliert, wenn man das Zwischenprodukt isolieren will.

$Kp_{0.04}$ = 170-180 °C, schwach gelbes, viskoses Öl.

Ausbeute : 95 % d. Th.

3. Stufe : Alkylierung

Die obige gewaschene Butanollösung wird mit 0,3 l Wasser und 0,5 Mol $K_2CO_3$ (70 g) versetzt, und unter Rühren tropft man bei maximal 45 °C, die Umsetzung ist exotherm, 1,1 Mol Dimethylsulfat = 104 ml zu. Man spült mit 10 ml n-Butanol nach und erwärmt auf 70-80 °C. Die Vollständigkeit wird dünnschichtchromatographisch geprüft.

Der DMS-Überschuß wird mit 10 ml konz. $NH_3$ zerstört und die Wasserphase abgetrennt (unten). Man wäscht mit 0,3 l Wasser und destilliert über 3 g $CaCO_3$.

$Kp_{0,03}$ = 130-150 °C
Fp. 49,9-52,8 °C erstarrt langsam
Ausbeute : 200-220 g

Das Destillat wird in 0,8 l Wasser und 1 Mol Essigsäure gelöst, über A-Kohle filtriert und das Hydrochlorid mit Salzsäure bei 80-90 °C bei pH = 4 gefällt. Bei RT saugt man ab und wäscht mit Aceton.

Fp. 232-235 °C (rein weiße Plättchen)
Ausbeute : 80 %

## Beispiel 10

N-[2(2-Hydroxyäthyl)-aminoäthyl]
(2-amino-3,5-dibrombenzyl)-amindihydrochlorid ($C_{11}H_{19}Br_2Cl_2N_3O$)

In 0,1 l Glycol löst man 0,5 Mol KOH, gibt 0,5 Mol Aminoäthyläthanolamin, 20 g Pottasche und 0,4 l Butanol hinzu. Bei 110-115 °C fügt man 0,5 Mol 3,5-Dibrom-N'-methansulfonylanthranilsäurehydrazid analog Beispiel 5 hinzu, arbeitet nach Beispiel 5 auf und reduziert analog.

Die Butanol-Phase wird nach Klärfiltration mit Salzsäure bis pH = 2 angesäuert und völlig eingeengt. Den Rückstand nimmt man in 0,5 l Methanol und 20 ml Salzsäure auf, saugt ab und kristallisiert aus 0,2 l Wasser um. Fp. 224-225,8 °C

Ausbeute : 112 g = 52 % d. Th.

| | | | | |
|---|---|---|---|---|
| Ber. : | C 30,02 | H 4,35 | Hal 52,44 | N 9,55 |
| Gef. : | C 30,00 | H 4,27 | Hal 51,91 | N 9,57 |

**Patentansprüche**

1. Verfahren zur Herstellung von 2-Amino-3,5-dibrombenzylaminen der allgemeinen Formel

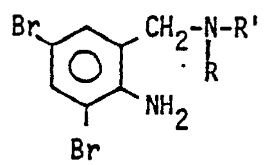

(I)

in der R für Wasserstoff oder $C_{1-3}$-Alkyl steht und R' einen gegebenenfalls substituierten aliphatischen Rest mit 1 bis 5 Kohlenstoffatomen, einen gegebenenfalls substituierten cycloaliphatischen Rest mit 5 oder 6 Kohlenstoffatomen oder eine Phenylgruppe, die gegebenenfalls substituiert ist, bedeutet, sowie deren physiologisch verträgliche Salze mit anorganischen oder organischen Säuren, dadurch gekennzeichnet, daß man

a) einen Anthranilsäurealkylester der allgemeinen Formel

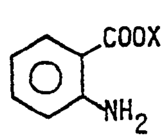

(II)

worin X für eine Niedrigalkylgruppe steht, in Gegenwart eines Oxidationsmittels zu dem 3,5-Dibromanthranilsäureester III

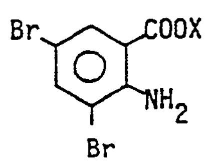

(III)

bromiert,

b) den enststandenen 3,5-Dibromanthranilsäureester III mit Hydrazin zum Hydrazid der allgemeinen Formel

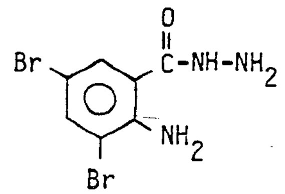

(IV)

umsetzt,

c) das erhaltene Hydrazid IV mit einem Sulfohalogenid zum N-(2-Amino-3,5-dibrombenzyl)-N'-sulfonylhydrazid der Formel

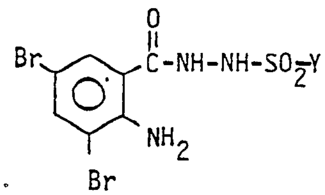

(V)

worin Y ein aliphatischer oder aromatischer Rest ist, umsetzt,

d) das erhaltene Sulfonylhydrazid V im alkalischen Medium mit einem primären Amin der allgemeinen Formel

$$H_2N—R' \qquad (VI)$$

worin R' die oben angegebene Bedeutung hat, zu der Schiff'schen Base VII

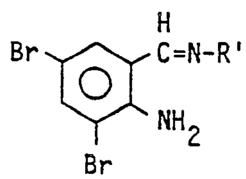

(VII)

umsetzt,

e) die erhaltene Schiff'sche Base VII zu der Verbindung der allgemeinen Formel I hydriert und gegebenenfalls

f) zur Herstellung einer Verbindung, bei der R für $C_{1-3}$-Alkyl steht, mit einem Alkylierungsmittel alkyliert und gegebenenfalls

g) die in Stufe e) oder f) erhaltene Verbindung in an sich bekannter Weise in ihr Salz überführt.

2. Verfahren nach Anspruch 1, dadurch gekennzeichnet, daß R für Wasserstoff steht und R' einen gegebenenfalls substituierten cycloaliphatischen Rest mit 5 oder 6 Kohlenstoffatomen bedeutet.

3. Verfahren nach Anspruch 2, dadurch gekennzeichnet, daß R' einen gegebenenfalls substituierten Cyclohexylrest bedeutet.

4. Verfahren nach Anspruch 3, dadurch gekennzeichnet, daß R' einen Hydroxycyclohexylrest bedeutet.

5. Verfahren nach Anspruch 1, dadurch gekennzeichnet, daß R für Methyl steht und daß R' einen Cyclohexylrest bedeutet.

6. Verfahren nach Anspruch 1, dadurch gekennzeichnet, daß man in Stufe a) Anthranilsäuremethylester mit 1 Mol Brom in Gegenwart von Wasserstoffperoxid, Wasser und einem mit Wasser nicht mischbaren Lösungsmittel umsetzt.

7. Verfahren nach Anspruch 1, dadurch gekennzeichnet, daß man in Stufe c) Dibromanthranilsäurehydrazid mit Methansulfonylchlorid umsetzt.

8. Verfahren nach Anspruch 1, dadurch gekennzeichnet, daß man in Stufe d) Methansulfonylhydrazid mit Methylglykol in Gegenwart von Kaliumcarbonat und dem primären Amin bei Temperaturen von 70 bis 150 °C umsetzt.

9. Verfahren nach Anspruch 1, dadurch gekennzeichnet, daß man in Stufe d) als Lösungsmittel Butanol oder einen homologen Alkohol verwendet und die Bildung des Benzylamins in einem Reaktionsschritt ohne Isolierung der Schiff'schen Base vornimmt.

10. Verfahren nach Anspruch 1, dadurch gekennzeichnet, daß man die Bildung des Benzylaminsbei konstantem pH-Wert in Gegenwart eines Puffers vornimmt.

**Claims**

1. A process for the preparation of 2-amino-3,5-dibromobenzylamines corresponding to the following general formula

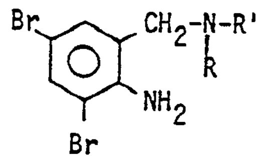

(I)

in which R represents hydrogen or $C_1$-$C_3$ alkyl and R' represents an optionally substituted $C_1$-$C_5$ aliphatic radical, an optionally substituted $C_5$ or $C_6$ cycloaliphatic radical or an optionally substituted phenyl group, and physiologically compatible salts thereof with inorganic or organic acids, characterized in that

a) an anthranilic acid alkyl ester corresponding to the following general formula

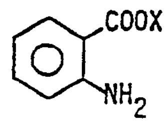

(II)

in which X is a lower alkyl group, is brominated in the presence of an oxidizing agent to form the 3,5-dibromoanthranilic acid ester III

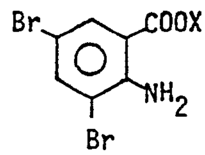

(III)

b) the 3,5-dibromoanthranilic acid ester III is reacted with hydrazine to form a hydrazide corresponding to the following general formula

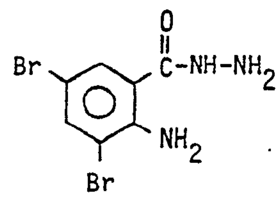

(IV)

c) the hydrazide IV obtained is reacted with a sulfohalide to form N-(2-amino-3,5-dibromobenzyl)-N'-sulfonyl hydrazide corresponding to the following formula

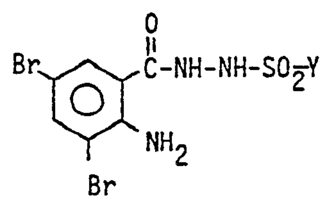

(V)

in which Y is an aliphatic or aromatic radical,

d) the sulfonylhydrazide V obtained is reacted in alkaline medium with a primary amine corresponding to the following general formula

$H_2N—R'$ (VI)

in which R' is a defined above, to form the Schiff's base VII

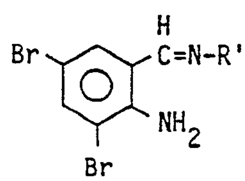

(VII)

e) the Schiff's base VII obtained is hydrogenated to the compound of general formula I and, optionally,

f) to prepare a compound in which R is $C_1$-$C_3$ alkyl, is alkylated with an alkylating agent and, optionally,

g) the compound obtained in step e) or step f) is converted into its salt in known manner.

2. A process as claimed in Claim 1, characterized in that R represents hydrogen and R′ represents an optionally substituted cycloaliphatic radical containing 5 or 6 carbon atoms.

3. A process as claimed in Claim 2, characterized in that R′ represents an optionally substituted cyclohexyl radical.

4. A process as claimed in Claim 3, characterized in that R′ represents a hydroxycyclohexyl radical.

5. A process as claimed in Claim 1, characterized in that R represents methyl and R′ represents a cyclohexyl radical.

6. A process as claimed in Claim 1, characterized in that in step a) anthranilic acid methyl ester is reacted with 1 mole bromine in the presence of hydrogen peroxide, water and a water-immiscible solvent.

7. A process as claimed in Claim 1, characterized in that in step c) dibromoanthranilic acid hydrazide is reacted with methane sulfonyl chloride.

8. A process as claimed in Claim 1, characterized in that in step d) methane sulfonyl hydrazide is reacted with methyl glycol in the presence of potassium carbonate and the primary amine at temperatures of from 70 to 150 °C.

9. A process as claimed in Claim 1, characterized in that in step d) butanol or a homologous alcohol is used as solvent and formation of the benzylamine is carried out in a single reaction step without isolation of the Schiff's base.

10. A process as claimed in Claim 1, characterized in that formation of the benzylamine is carried out at a constant pH-value in the presence of a buffer.

**Revendications**

1. Procédé de préparation de 2-amino-3,5-dibromobenzylamines répondant à la formule générale

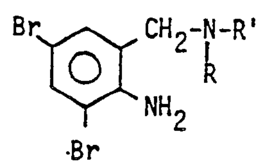

(I)

dans laquelle R représente l'hydrogène ou un groupe alcoyle comportant 1 à 3 atomes de carbone et R′ représente un reste aliphatique comportant 1 à 5 atomes de carbone, éventuellement substitué, un reste cyclo-aliphatique comportant 5 ou 6 atomes de carbone, éventuellement substitué, ou un groupe phényle éventuellement substitué, ainsi que de leurs sels physiologiquement compatibles avec des acides inorganiques ou organiques, caractérisé en ce que :

a) l'on brome un ester alcoylique de l'acide anthranilique répondant à la formule générale

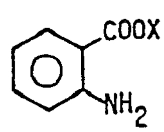

(II)

dans laquelle X représente un groupe alcoyle inférieur, en présence d'un oxydant, ce qui aboutit à l'ester III de l'acide 3,5-dibromanthranilique

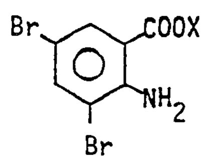

(III)

b) on fait réagir l'ester 3,5-dibromanthranilique III obtenu sur de l'hydrazine, ce qui aboutit à un hydrazide répondant à la formule générale

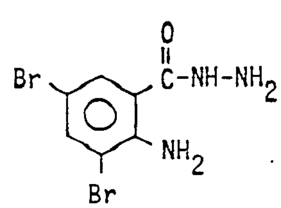

(IV)

c) on fait réagir l'hydrazide IV obtenu sur un sulfo-halogénure, ce qui aboutit au N-(2-amino-3,5-dibromobenzyl)-N'-sulfonylhydrazide répondant à la formule

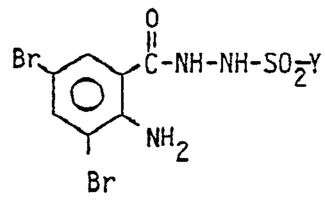

(V)

dans laquelle Y est un reste aliphatique ou aromatique,

d) on fait réagir le sulfonylhydrazide V obtenu, en milieu alcalin, sur une amine primaire répondant à la formule générale

$$H_2N—R' \quad (VI)$$

dans laquelle R' a la signification indiquée précédemment, ce qui aboutit à la base de Schiff VII

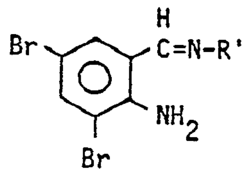

(VII)

e) on hydrogène la base de Schiff VII obtenu pour aboutir au composé répondant à la formule générale I, et éventuellement

f) pour préparer un composé dans lequel R représente un groupe alcoyle comportant 1 à 3 atomes de carbone, on l'alcoyle au moyen d'un agent d'alcoylation et éventuellement

g) on transforme, de façon connue en soi, le composé obtenu au stade e) ou f) en son sel.

2. Procédé selon la revendication 1, caractérisé en ce que R représente l'hydrogène et R' un reste cycloaliphatique comportant 5 ou 6 atomes de carbone, éventuellement substitué.

3. Procédé selon la revendication 2, caractérisé en ce que R' représente un reste cyclohexyle éventuellement substitué.

4. Procédé selon la revendication 3, caractérisé en ce que R' représente un reste hydroxycyclohexyle.

5. Procédé selon la revendication 1, caractérisé en ce que R représente le groupe méthyle, et en ce que R' représente un reste cyclohexyle.

6. Procédé selon la revendication 1, caractérisé en ce qu'au stade a), on fait réagir l'ester méthylique de l'acide anthranilique (anthranilate de méthyle) sur une mole de brome, en présence d'eau oxygénée, d'eau et d'un solvant non miscible à l'eau.

7. Procédé selon la revendication 1, caractérisé en ce qu'on fait réagir, au stade c), l'hydrazide de l'acide dibromanthranilique sur le chlorure de méthane-sulfonyle.

8. Procédé selon la revendication 1, caractérisé en ce qu'au stade d), on fait réagir le méthane-sulfonyl-hydrazide sur du méthylglycol, en présence de carbonate de potassium et de l'amine primaire à des températures de 70 à 150 °C.

9. Procédé selon la revendication 1, caractérisé en ce qu'on utilise comme solvant, au stade d), du butanol ou un alcool homologue, et l'on effectue la formation de la benzylamine au cours d'un stade réactionnel sans isolement de la base de Schiff.

10. Procédé selon la revendication 1, caractérisé en ce qu'on effectue la formation de la benzylamine à une valeur de pH constante, en présence d'un tampon.